(19) Europäisches Patentamt
European Patent Office
Office européen des brevets

(11) Veröffentlichungsnummer: **0 525 626 B1**

(12) # EUROPÄISCHE PATENTSCHRIFT

(45) Veröffentlichungstag der Patentschrift: **14.06.95**

(51) Int. Cl.$^{6}$: **C07C 69/96**, G03F 7/004

(21) Anmeldenummer: **92112560.5**

(22) Anmeldetag: **22.07.92**

(54) **Verbindungen mit säurelabilen Schutzgruppen und damit hergestelltes positiv arbeitendes strahlungsempfindliches Gemisch.**

(30) Priorität: **31.07.91 DE 4125258**

(43) Veröffentlichungstag der Anmeldung:
**03.02.93 Patentblatt 93/05**

(45) Bekanntmachung des Hinweises auf die Patenterteilung:
**14.06.95 Patentblatt 95/24**

(84) Benannte Vertragsstaaten:
**DE FR GB**

(56) Entgegenhaltungen:
**EP-A- 0 031 566**
**EP-A- 0 355 581**
**DE-A- 2 342 068**

(73) Patentinhaber: **HOECHST AKTIENGESELLSCHAFT**

**D-65926 Frankfurt (DE)**

(72) Erfinder: **Cabrera, Ivan, Dr.**
**Am Hermannsberg 21**
**W-6080 Gross-Gerau (DE)**
Erfinder: **Spiess, Walter, Dr.**
**Rheingaustrasse 20**
**W-6110 Dieburg (DE)**
Erfinder: **Pawlowski, Georg, Dr.**
**Fritz-Kalle-Strasse 34**
**W-6200 Wiesbaden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Dokumente EP-A-0 355 581, DE-A-2 342 068 und EP-A-0 031 566 beschreiben Acetale und deren Verwendung in lichtempfindlichen Gemischen.

Die Erfindung betrifft Verbindungen mit säurelabilen Schutzgruppen, Verfahren zu ihrer Herstellung sowie positiv arbeitende strahlungsempfindliche Gemische, die diese Verbindungen als Lösungsinhibitoren enthalten. Sie betrifft weiterhin ein damit hergestelltes Aufzeichnungsmaterial, das für Photoresists, elektrische Bauteile und Druckplatten sowie zum Formteilätzen geeignet ist.

In der industriellen Herstellung mikroelektronischer Bauteile werden derzeit mehrere lithographische Techniken eingesetzt, wobei an die dabei verwendeten Photoresistgemische jeweils verschiedene Anforderungen gestellt werden. So finden in der g-line-Lithographie, bei der Strahlung einer Wellenlänge von 436 nm eingesetzt wird, in der Regel klassische Diazonaphthochinon-Novolak-Photoresists Verwendung. Eine neuere Entwicklung ist die i-line-Lithographie, bei der Strahlung einer Wellenlänge von 365 nm genutzt wird. Diese Technik erlaubt es, Details einer Maskenvorlage in einer verbesserten Auflösung bis herab zu 0,5 $\mu$m abzubilden. Neuere Modifikationen, wie die Phase-Shifting-Mask-Technologie, erlauben eine weitere Verkleinerung der abzubildenden Strukturen bis herab zu etwa 0,35 $\mu$m und sogar noch feiner. Eine noch weiter gesteigerte Auflösung werden in Zukunft die "UV-2"-Photoresists gestatten. Hierbei unterscheidet man zwischen zwei Ansätzen: der UV-2 Breitbandbestrahlung (240 bis 260 nm) und der Bestrahlung mit KrF-Excimer-Lasern (248 nm).

Wie bereits aus dem Vorangehenden ersichtlich, ist die Grenze der Auflösung durch die Wellenlänge der verwendeten Strahlung vorgegeben. Die stetige Verkleinerung der Strukturdimensionen bis weit in den Submikron-Bereich, insbesondere bei Mikrochips, erfordert daher veränderte Lithographie-Techniken. Wegen ihrer kurzen Wellenlänge eignen sich hier insbesondere energiereiche UV-Strahlung, Elektronen- oder Röntgenstrahlung. Eine Übersicht über die Ansprüche, die an die jeweils verwendeten strahlungsempfindlichen Gemische gestellt werden, ist in dem Aufsatz von C.G. Willson, "Organic Resist Materials- Theory and Chemistry" (Introduction to Microlithography, Theory, Materials, and Processing; Herausgeber: L.F. Thompson, C.G. Willson, M.J. Bowden; ACS Symp. Ser., 219 [1983] S. 87, American Chemical Society, Washington) zu finden. Es besteht daher ein verstärkter Bedarf an strahlungsempfindlichen Gemischen, die in den fortgeschrittenen Technologien, insbesondere der mid- und deep-UV-, der Elektronen- und Röntgenstrahl-Lithographie, einsetzbar sind und vorzugsweise darüber hinaus in einem weiten Spektralbereich empfindlich und somit auch in der konventionellen UV-Lithographie einsetzbar sind.

Ein vielfach verwendetes positiv arbeitende strahlungsempfindliches Gemisch zur Herstellung strahlungsempfindlicher Aufzeichnungsmaterialien enthält ein o-Chinondiazid-Derivat und ein in wäßrig-alkalischen Lösemitteln lösliches Bindemittel, z. B. einen Novolak oder ein Polyhydroxystyrol. Die Empfindlichkeit der Aufzeichnungsmaterialien gegenüber UV-Strahlung, insbesondere hochenergetischer, kurzwelliger Strahlung, beispielsweise Licht eines KrF-Excimer-Lasers mit einer Wellenlänge von 248 nm oder Elektronenstrahlung, ist jedoch allgemein unzureichend.

Eine verbesserte Empfindlichkeit weisen positiv arbeitende strahlungsempfindliche Gemische auf, in denen ein Photoinitiator durch Einwirkung von aktinischer Strahlung eine Säure bildet. Diese Säure spaltet in einer Folgereaktion ein säurespaltbares Material und macht es dadurch in wäßrig-alkalischen Entwicklern löslich.

Es ist ferner bekannt, daß Verbindungen mit phenolischen OH-Gruppen durch tert.-Butoxycarbonylgruppen "maskiert" werden können. Durch Säuren wird dieses Derivat in die phenolische Ausgangsverbindung, Kohlendioxid und Isobuten gespalten. Auch solche Verbindungen können als lichtempfindliche Löslichkeitsinhibitoren genutzt werden.

Strahlungsempfindliche Gemische mit säurespaltbaren Löslichkeitsinhibitoren benötigen immer eine geringe Menge einer Verbindung, die bei Bestrahlung Säure bildet, die wiederum die Spaltung der oben genannten Materialien bewirkt. Als photolytische Säurebildner wurden bisher insbesondere Onium-Salze, wie Diazonium-, Phosphonium-, Sulfonium- und Jodonium-Salze von nicht nucleophilen Säuren, wie $HSbF_6$, $HAsF_6$, oder $HPF_6$ verwendet. Daneben sind Halogenverbindungen, insbesondere Trichlormethyltriazin-Derivate oder Trichlormethyloxadiazol-Derivate, o-Chinondiazidsulfochloride, o-Chinondiazid-4-sulfonsäureester, Organometall-Organohalogen-Kombinationen, Bis(sulfonyl)-diazomethane, Sulfonyl-carbonyl-diazomethane oder Nitrobenzyltosylate empfohlen.

Derartige Gemische mit z. T. hohen Empfindlichkeiten gegenüber aktinischer Strahlung werden als photokatalytische 3-Komponentensysteme bezeichnet, da sie als wesentliche Bestandteile ein polymeres, in wäßrig-alkalischen Lösungen lösliches Bindemittel - zumeist ein phenolisches Harz -, eine photoaktive Verbindung und einen säurespaltbaren Löslichkeitsinhibitor enthalten. Unter diesen Gemischen konnten sich in der Praxis besonders solche durchsetzen, die als säurelabile Komponente Verbindungen mit Acetalgruppen aufweisen, da

diese einerseits ausreichende Spaltbarkeit, andererseits aber auch ausreichende Lagerstabilität, insbesondere in gelöster Form, miteinander kombinieren. Das Acetal muß dabei unter anderem ein weitgehend hydrophobes molekulares Gerüst aufweisen, damit es als Lösungsinhibitor fungieren kann. Acetale mit freien phenolischen Hydroxylgruppen sind als Lösungsinhibitoren völlig ungeeignet, da sie eher die Löslichkeit in wäßrig-alkalischen Lösungen verstärken.

Darüber hinaus ist allgemein zu beobachten, daß das Prozeßfenster, d. h. der Spektralbereich der Durchlässigkeit, für die Belichtung dieser Gemische sehr schmal und häufig nicht eindeutig reproduzierbar ist, was zu ungenauen Vorlagewiedergaben führt. Das unzureichend schmale Prozeßfenster äußert sich insbesondere in einer starken Abhängigkeit der Qualität der Bildwiedergabe von der Zeitdifferenz zwischen Belichtung und Entwicklung, der sogenannten "delay time". Die Ursachen dieser Verschlechterung der Bildwiedergabe sind nicht im einzelnen bekannt oder nur mangelhaft untersucht worden. Grundsätzlich ist davon auszugehen, daß Diffusionsvorgänge, die zu diesem Verhalten führen, nicht einfach steuerbar sind. Es läßt sich jedoch vermuten, daß es während der Trocknung des Gemisches auf einem Substratmaterial zu einer partiellen Verdampfung des Photoinitiators oder der säurelabilen Verbindung oder zu einer Entmischung der einzelnen Gemischbestandteile kommt, was besonders häufig bei säurelabilen Verbindungen mit geringer Löslichkeit in den üblichen Beschichtungslösemitteln beobachtet wird.

Der entscheidende Nachteil der bekannten, Acetalgruppen aufweisenden Verbindungen beruht darin, daß die durch ihre Spaltung resultierende Löslichkeitsdifferenzierung zwischen belichteten und unbelichteten Bildbereichen im allgemeinen ungenügend ist. Als Ursache ist anzunehmen, daß entweder das als Lösungsinhibitor eingesetzte Acetalderivat eine unzureichende Inhibierungseigenschaft aufweist und bei einer bildmäßigen Differenzierung neben den belichteten Bildstellen auch solche stark angegriffen und abgetragen werden, die nicht belichtet wurden, oder aber, daß die belichteten Bereiche keine ausreichende Löslichkeit aufweisen, um bei der Entwicklung eine bildmäßige Differenzierung zu ermöglichen. Das Problem ist, daß es mit den bekannten Verbindungen nicht gelingt, ein Material bereitzustellen, das eine ausreichend große Löslichkeitsdifferenz zwischen belichteten und unbelichteten Bereichen hervorruft. Während dieser Effekt bei den gemäß dem Stand der Technik verwendeten Novolakharzen im allgemeinen noch akzeptabel ist, beobachtet man bei Verwendung von anderen Polymeren, daß die bekannten Acetalderivate praktisch keinerlei Inhibierungswirkung mehr zeigen und damit eine praxisgerechte Bilddifferenzierung nicht mehr erlauben.

Bei der säurekatalysierten Spaltung von 1 mol des Acetals entstehen 1 mol des entsprechenden Aldehyds und 2 mol Alkohol. Der Alkohol trägt im allgemeinen zur verbesserten Löslichkeit in alkalischen Entwicklern bei. Der Aldehyd hingegen vermindert die Löslichkeit, so daß er häufig durch einen zusätzlichen Backschritt aus dem Gemisch verdampft wird. Es ist jedoch vorteilhafter, den Aldehyd in der aus dem Gemisch bestehenden Schicht zu belassen, da er nur in nicht kontrollierbarer Weise verdampft werden kann, insbesondere bei unterschiedlichen Schichtdicken.

Dies führt zu nicht reproduzierbaren Ergebnissen bezüglich Empfindlichkeit und Entwicklungsverhalten.

In beiden Fällen werden daher keine optimalen Ergebnisse erhalten. Muß der lösungsinhibierende, durch Spaltung erzeugte Aldehyd aus dem Gemisch herausgedampft werden, ist ein zusätzlicher Verarbeitungsschritt notwendig, um die bestmögliche Löslichkeit der belichteten Bereiche zu erhalten. Bleibt der inhibierend wirkende Aldehyd in dem Gemisch, so wird keine praxisgerechte Differenzierung der Löslichkeit zwischen belichteten und unbelichteten Schichtbereichen erreicht. Dies hat wiederum dann besonders nachteilige Folgen, wenn das Gemisch in einem Aufzeichnungsmaterial verwendet wird. In diesem Fall müssen längere Belichtungs- und Entwicklungszeiten in Kauf genommen werden, und das erhaltene Reliefbild weist infolge der unzureichenden Löslichkeitsdifferenzierung zwischen belichteten und unbelichteten Bereichen Schwächen im Kontrast, im Strukturprofil und im Dunkelabtrag auf.

Es bestand daher die Aufgabe, Verbindungen bereitzustellen, die einerseits die Löslichkeit in Wasser wirksam herabsetzen, deren Spaltprodukte jedoch in wäßrig-alkalischen Entwicklern sehr gut löslich sind, den Entwicklungsprozeß in den belichteten Bereichen unterstützen und gleichzeitig eine geringe Flüchtigkeit aufweisen, damit sie auch bei einer thermischen Nachbehandlung nicht aus dem Gemisch herausdampfen, und somit die Eigenschaften des strahlungsempfindlichen Gemischs kontrollierbar bleiben.

Gelöst wird die Aufgabe durch neue, substituierte Acetale und Ketale der folgenden allgemeinen Formel I, die unter dem Einfluß ausreichend starker Säure gespalten werden und dabei einen hydroxysubstituierten aromatischen Aldehyd oder ein hydroxysubstituiertes aromatisches Keton freisetzen.

Gegenstand der vorliegenden Erfindung sind daher Verbindungen der allgemeinen Formel I

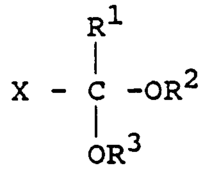

(I)

worin

X ein Phenyl-, [1]Naphthyl- oder [2]-Naphthylrest ist, der jeweils mit mindestens einer tert.-Butoxycarbonyloxygruppe substituiert ist und gegebenfalls einen oder mehr als einen zusätzlichen Substituenten aufweist,

$R^1$ für ein Wasserstoffatom, einen $(C_1\text{-}C_6)$Alkyl-, einen $(C_6\text{-}C_{10})$-Arylrest oder einen der Reste X steht und

$R^2$ und $R^3$ gleich oder verschieden sind und einen $(C_1\text{-}C_{12})$Alkylrest, in dem gegebenenfalls bis zu drei Methylengruppen durch Brückenglieder, die mindestens ein Heteroatom aufweisen, wie -O-, -S-, $\text{-NR}^4\text{-}$, -CO-, -CO-O-, -CO-NH-, -O-CO-NH-, -NH-CO-NH-, -CO-NH-CO-, $\text{-SO}_2\text{-}$, $\text{-SO}_2\text{-O-}$, oder $\text{-SO}_2\text{-NH-}$, ersetzt sind, einen $(C_3\text{-}C_{12})$-Alkenyl-, $(C_3\text{-}C_{12})$Alkinyl, $(C_4\text{-}C_{12})$Cycloalkyl-, $(C_4\text{-}C_{12})$-Cycloalkenyl- oder $(C_8\text{-}C_{16})$-Aralkylrest, in dem gegebenenfalls bis zu drei Methylengruppen des aliphatischen Teils durch Brückenglieder der oben genannten Art ersetzt sind und der im aromatischen Teil durch Fluor-, Chlor- oder Bromatome, durch $(C_1\text{-}C_4)$Alkyl-, $(C_1\text{-}C_4)$Alkoxy-, Nitro-, Cyano- oder tert.-Butoxycarbonyloxygruppen substituiert sein kann, bedeuten, wobei

$R^4$ für einen Acyl-, insbesondere einen $(C_1\text{-}C_6)$Alkanoylrest, steht.

Die gegebenenfalls zusätzlich in den Resten X vorkommenden Substituenten sind bevorzugt Fluor-, Chlor-, Brom- oder Jodatome, Nitro-, Cyano- oder Carboxygruppen, $(C_1\text{-}C_9)$-, besonders bevorzugt $(C_1\text{-}C_6)$Alkylreste, in denen gegebenenfalls bis zu drei, besonders bevorzugt bis zu zwei, Methylengruppen durch Brückenglieder der oben genannten Art ersetzt sind, Phenylreste, die wiederum substituiert sein können, insbesondere mit tert.-Butoxycarbonylgruppen, $(C_1\text{-}C_4)$Alkylresten, $(C_1\text{-}C_4)$-Alkoxyresten und/oder Halogen-atomen, sowie $(C_8\text{-}C_{12})$-, besonders bevorzugt $(C_8\text{-}C_{10})$Aralkylreste, in denen gegebenenfalls bis zu zwei Methylengruppen durch Brückenglieder der obengenannten Art ersetzt sind, $(C_6\text{-}C_{10})$Aryloxyreste oder $(C_7\text{-}C_{10})$-Aralkoxyreste.

Die Heteroatome enthaltenden Brückenglieder in den Alkyl- und Aralkylresten können innerhalb der Alkylkette auftreten oder das Verbindungsglied zum Rest X darstellen.

Allgemein bevorzugt sind Verbindungen der allgemeinen Formel I, in denen X für einen substituierten Phenylrest steht. Besonders bevorzugt sind Verbindungen der allgemeinen Formel I, in denen $R^1$ darüber hinaus ein Wasserstoffatom bedeutet.

Die erfindungsgemäßen Verbindungen stellen Acetale bzw. Ketale dar, die ausgeprägt hydrophob und daher als Lösungsinhibitoren geeignet sind, während die Produkte, die aus der sauer katalysierten Spaltung dieser Verbindungen hervorgehen, außerordentlich hydrophil sind und daher als Lösungspromotoren wirken.

Gegenstand der Erfindung ist weiterhin ein Verfahren zur Herstellung der Verbindungen der allgemeinen Formel I, in dem man eine Carbonylverbindung der allgemeinen Formel II

$X\text{-CO-}R^1$ (II)

worin X und $R^1$ die oben genannten Bedeutungen besitzen, mit einem niedrigsiedenden Alkohol in Gegenwart eines Katalysators zu den entsprechenden Acetalen bzw. Ketalen der allgemeinen Formel III

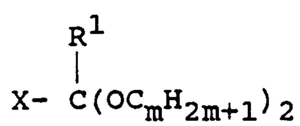

(III)

umsetzt. Der niedrigsiedende Alkohol ist vorzugsweise Methanol oder Ethanol, das heißt m ist vorzugsweise 1 oder 2.

Um das bei der Umsetzung gebildete Reaktionswasser irreversibel aus dem Reaktionsgemisch zu entfernen, enthält die Mischung zweckmäßig ein geeignetes wasserentziehendes Mittel, z. B. ein Trocknungsmittel.

Die erfindungsgemäßen Verbindungen der allgemeinen Formel I werden dann durch Umsetzung mit den höhersiedenden Alkoholen $R^2$-OH und/oder $R^3$-OH in Gegenwart eines Katalysators gewonnen.

Die Verbindungen der allgemeinen Formel II lassen sich aus den entsprechenden, mit Hydroxygruppen substituierten aromatischen Aldehyden bzw. Ketonen in an sich bekannter Weise (F. Houlihan, et. al., Can. J. Chem., 63, 153 (1985)) durch Umsetzung mit tert.-Butoxycarbonylchlorid oder

Pyrokohlensäure-di-tert.-butylester herstellen.

Besonders bevorzugte Ausgangsmaterialien sind hydroxysubstituierte aromatische Aldehyde, wie 2-, 3- und 4-Hydroxybenzaldehyd, 2,3-, 2,4-, 2,5- und 3,4-Dihydroxybenzaldehyd, 2,3,4-Trihydroxybenzaldehyd, 4-Hydroxy-3-methylbenzaldehyd, 2-Hydroxy-4- und -5-methoxybenzaldehyd, 3-Hydroxy-4-methoxybenzaldehyd, 4-Hydroxy-3-methoxybenzaldehyd, 3,5-Dimethyl-4-hydroxybenzaldehyd, 3,4-Dimethoxy-5-hydroxybenzaldehyd, 3-Ethoxy-4-hydroxybenzaldehyd, 2-Hydroxy-5-nitrobenzaldehyd, 3-Hydroxy-4-nitrobenzaldehyd, 4-Hydroxy-3-nitrobenzaldehyd, 5-Hydroxy-2-nitrobenzaldehyd oder 2-Hydroxy-1-naphthaldehyd.

Diese Aldehyde sind käuflich, während sich andere, als Vorprodukte ebenfalls geeignete aromatische Hydroxyaldehyde oder -ketone nach den unterschiedlichsten Methoden im allgemeinen auf einfache Weise herstellen lassen (siehe Houben Weyl, Methoden der organischen Chemie, Bd. 7/1).

Um eine Spaltung der tert.-Butoxycarbonyloxygruppe zu vermeiden, wird die Acetalisierung bzw. Ketalisierung der Verbindungen der Formel II mit den niedrigsiedenden Alkoholen in Gegenwart eines speziellen Katalysators ausgeführt. Als geeignet haben sich Katalysatoren vom Typ der Rhodiumphosphin-Komplexe erwiesen. Bevorzugt sind Katalysatoren vom Typ $RhCl_3$(triphos). Besonders bevorzugt wird der Katalysator $RhCl_3[CH_3C(CH_2PPh_2)_3]$ eingesetzt.

Die erforderliche Menge des Katalysators liegt im allgemeinen zwischen 0,1 mg und 10 mg pro 1 mmol Aldehyd, bevorzugt bei 0,5 bis 1 mg Katalysator pro 1 mmol Aldehyd. Diese Katalysatoren sind von J. Ott et al. in J. Organomet. Chem., 291, 89 (1985) und Tetrahedr. Lett., 30, 6151 (1989)) beschrieben worden.

Die Acetalisierung bzw. Ketalisierung erfolgt zweckmäßig bei Temperaturen zwischen 0 °C und 100 °C. Bevorzugt werden Temperaturen zwischen 10 °C und 50 °C, insbesondere Raumtemperatur. Die Reaktionszeit liegt zwischen 20 Minuten und 48 Stunden, insbesondere zwischen 4 und 16 Stunden.

Die Umwandlung kann in Gegenwart eines unter den Reaktionsbedingungen inerten Lösemittels durchgeführt werden. Geeignet sind besonders aromatische Kohlenwasserstoffe, wie Toluol und Xylol. Das Lösemittel wird in einem 0,5 bis 50fachen Überschuß zugesetzt.

Um das entstehende Reaktionswasser irreversibel zu entfernen, wird dem Reaktionsgemisch der dem Alkohol entsprechende Orthoameisentrialkylester, bevorzugt Orthoameisensäuretrimethyl- oder -triethylester, zugesetzt.

Das Gemisch wird nach bekannten Methoden aufgearbeitet. Es werden Ausbeuten von mehr als 90% erhalten.

Die Acetalisierung bzw. Ketalisierung der Verbindungen der allgemeinen Formel II kann auch in Gegenwart von anderen neutralen oder sehr schwach sauren Katalysatoren, wie sauren Austauscherharzen oder organischen Säuren, durchgeführt werden. Diese Katalysatoren führen jedoch zu einer unvollständigen Umsetzung oder zur Umesterung und damit zu Schwierigkeiten in der Aufarbeitung sowie zur Verminderung der Produktausbeute und -reinheit.

Mit den oben genannten Rhodium-Katalysatoren können die Aldehyde bzw. Ketone der allgemeinen Formel II auch direkt mit den höhersiedenden Alkoholen $R^2$-OH und/oder $R^3$-OH umgesetzt werden. Es ergeben sich jedoch Probleme bei der Aufarbeitung, die bei dem oben geschilderten zweistufigen Verfahren nicht auftreten.

Die erfindungsgemäßen Verbindungen der allgemeinen Formel I werden dann aus den Verbindungen der Formel III in einer zweiten Stufe durch Umsetzung mit einer stöchiometrischen Menge an hochsiedenden Alkoholen $R^2$-OH und/oder $R^3$-OH in einem inerten Lösemittel, wie Toluol und Xylol, in Gegenwart eines sauren Katalysators gewonnen. Um auch hier eine Zersetzung der tert.-Butoxycarbonyloxygruppe zu vermeiden, werden nur schwach saure, im Reaktionsgemisch unlösliche Katalysatoren verwendet. Bevorzugt wird als Katalysator Kaliumhydrogensulfat oder ein saures Ionenaustauscherharz verwendet.

Pro 1 mmol eingesetzten Acetals oder Ketals werden 1 bis 100 mg Katalysator verwendet. Bevorzugt ist ein Mengenverhältnis von 5 bis 10 mg Katalysator/mmol Carbonylderivat. Zur Erhöhung der Reaktionsgeschwindigkeit wird die Umsetzung bei Temperaturen von mehr als 75 °C durchgeführt, bevorzugt sind Temperaturen zwischen 100 und 150 °C.

Die Verbindungen der allgemeinen Formel I werden aus den Verbindungen der allgemeinen Formel III in einer Ausbeute von 75 bis 98% der theoretisch möglichen erhalten.

Die erfindungsgemäßen Verbindungen der allgemeinen Formel I eignen sich in hervorragender Weise zur Herstellung von positiv arbeitenden strahlungsempfindlichen Gemischen, die in strahlungsempfindlichen Schichten für Photoresists oder Druckplatten eingesetzt werden können.

Gegenstand der vorliegenden Erfindung ist weiterhin ein positiv arbeitendes strahlungsempfindliches Gemisch mit

a) einer Verbindung, die unter der Einwirkung von aktinischer Strahlung starke Säure bildet,

b) eine Verbindung mit mindestens eine C-O-C-Bindung, die durch die von der Verbindung a) gebildete Säure gespalten werden kann, und

c) ein in Wasser unlösliches, in wäßrig-alkalischer Lösung dagegen lösliches oder zumindest

quellbares Bindemittel,
das dadurch gekennzeichnet ist, daß die Verbindung b) eine Verbindung der allgemeinen Formel I ist.

Das erfindungsgemäße strahlungsempfindliche Gemisch zeichnet sich durch eine hohe Empfindlichkeit über einen weiten Spektralbereich aus. Es zeigt eine hohe thermische Stabilität und schafft die Möglichkeit, auch feinste Strukturen einer Vorlage detailgenau wiederzugeben.

Der Gehalt an säurespaltbarem Material b) in dem erfindungsgemäßen strahlungsempfindlichen Gemisch sollte bei 1 bis 60 Gew.-%, vorzugsweise bei 5 bis 50 Gew.-%, jeweils bezogen auf das Gesamtgewicht der festen Anteile des Gemisches, liegen.

Die erfindungsgemäßen Gemische können gegebenenfalls noch andere säurespaltbare Verbindungen enthalten. Dabei haben sich vor allem folgende Verbindungsklassen bewährt:

1. solche mit mindestens einer Orthocarbonsäureester- und bzw. oder -carbonsäureamidacetalgruppierung, wobei die Verbindungen auch polymeren Charakter haben und die genannten Gruppierungen als verknüpfende Elemente in der Hauptkette oder als seitenständige Substituenten auftreten können (DE-A 26 10 842 und 29 28 636),
2. Oligomer- oder Polymerverbindungen mit wiederkehrenden Acetal- und/oder Ketalgruppierungen in der Hauptkette (DE-C 23 06 248 und 27 18 254),
3. Verbindungen mit mindestens einer Enolether- oder N-Acyliminocarbonatgruppierung(EP-A 0 006 626 und 0 006 627),
4. cyclische Acetale oder Ketale von $\beta$-Ketoestern oder -amiden (EP-A 0 202 196),
5. Verbindungen mit Silylethergruppierungen (DE-A 35 44 165 und 36 01 264),
6. Verbindungen mit Silyl-enolethergruppierungen (DE-A 37 30 785 und 37 30 783),
7. Monoacetale bzw. Monoketale, deren Aldehyd- bzw. Ketonkomponente eine Löslichkeit im Entwickler zwischen 0,1 und 100 g/l aufweisen (DE-A 37 30 787),
8. Ether auf der Basis tertiärer Alkohole (US-A 4 603 101) und
9. Carbonsäureester und Carbonate tertiärer, allylischer oder benzylischer Alkohole [US-A 4 491 628 sowie von J. M. Fréchet et al., J. Imaging Sci. 30, 59-64 (1986)].

Diese Materialien können auch als Mischungen enthalten sein. Als zusätzliche säurespaltbare Komponente sind Materialien bevorzugt, die nur einem der obengenannten Typen entsprechen, darunter besonders bevorzugt solche mit mindestens einer durch Säure spaltbaren C-O-C-Bindung, d. h. diejenigen, die den Typen (1), (2), (7) und (9) angehören. Unter Typ (2) sind die polymeren Acetale hervorzuheben; von den säurespaltbaren Materialien des Typs (7) besonders diejenigen, die sich von Aldehyden bzw. Ketonen mit einem Siedepunkt über 150 °C, vorzugsweise über 200 °C, ableiten.

Den im erfindungsgemäßen strahlungsempfindlichen Gemisch enthaltenen säurespaltbaren Verbindungen b) werden photolytische Säurebildner a) zugesetzt, wozu sich nur eingeschränkt Onium-Salze, wie Diazonium-, Phosphonium-, Sulfonium- und Jodonium-Salze von nicht nucleophilen Säuren, z. B. von $HSbF_6$, $HAsF_6$, oder $HPF_6$ [J.V. Crivello, Polym. Eng. Sci., 23, 953 (1983)] eignen, da stark korrodierend wirkenden Säuren gebildet werden können. Bevorzugt sind photolytischen Säurebildner, die bei der Belichtung Sulfonsäuren bilden. Als Beispiele für solche Verbindungen seien 1,2-Disulfone, Bis-sulfonyl-diazomethan (DE-A 39 30 086) und Sulfonyl-carbonyl-diazomethan (DE-A 39 30 087) Nitrobenzylsulfonate (F.M. Houlihan et al., J. Photopolym. Sci. Techn., 3, 259, 1990; T. Yamaoka et al., J. Photopolym. Sci. Techn., 3, 275, 1990), Pyrogallolsulfonate (L. Schlegel et al., J. Photopolym. Sci. Techn., 3, 281, 1990) oder Iminosulfonate (M. Shirai et al., J. Photopolym. Sci. Techn., 3, 301, 1990) erwähnt. Besonders bevorzugt sind Onium-Salze, die Perfluorsulfonsäuren bilden, sowie die in der nicht vorveröffentlichten deutschen Patentanmeldung P 41 12 967.9 genannten 1-Sulfonyloxy-2-pyridone.

Die photolytischen Säurebildner werden dem Gemisch im allgemeinen mit einem Anteil von 0,2 bis 25 Gew-% zugesetzt. Bevorzugt sind Anteile von 0,5 bis 15 Gew.-%, besonders bevorzugt 1 bis 10 Gew.-%, bezogen auf das Gesamtgewicht der festen Anteile des Gemisches, wobei die Art der Komponenten a) bis c) das Verhältnis der verwendeten Verbindungen untereinander bestimmt.

Die photolytischen Säurebildner haben Absorptionsmaxima im Bereich zwischen 200 und 500 nm. Durch sie wird die spektrale Empfindlichkeit der erfindungsgemäßen Gemische festgelegt. Sie können so ausgewählt werden, daß man praxisgerechte Empfindlichkeiten im Bereich der technisch wichtigen Strahlungsguellen, z. B. g-line (436 nm), i-line (365 nm) oder UV2 (248 nm), erzielen kann. Die erfindungsgemäß verwendbaren säurespaltbaren Verbindungen (b) können dabei so ausgewählt werden, daß sie in diesen Bereichen praktisch keine Eigenabsorption aufweisen. Durch spektrale Sensibilisierung läßt sich der Empfindlichkeitsbereich der säurebildenden Verbindungen (a) so weit vergrößern, daß man auch Strahlungsguellen des sichtbaren oder kurzwelligen Bereichs der Röntgenstrahlung verwenden kann. Schließlich lassen sich auch andere Strahlungsguellen, wie Elektronen- oder Ionenstrahlen zur bildmäßigen Differenzierung des erfindungsgemäßen Gemisches einsetzen, insbesondere dann, wenn man hochaktive Säurebildner (EP-A 0 318 649) einsetzt.

Das erfindungsgemäße strahlungsempfindliche Gemisch enthält ferner mindestens ein polymeres, in Wasser unlösliches, in wäßrigen alkalischen Lösungen aber lösliches, zumindest aber quellbares Bindemittel c). Die Wahl des geeigneten Bindemittels richtet sich weitgehend nach der Verwendungsart. Das Bindemittel zeichnet sich im besonderen dadurch aus, daß es die Bestandteile des erfindungsgemäßen strahlungsempfindlichen Gemisches gut löst, eine wäßrig-alkalische Entwickelbarkeit erlaubt und insbesondere im Wellenlängenbereich der Einstrahlung von 190 bis 550 nm eine möglichst geringe Eigenabsorption, d.h. eine hohe Transparenz, aufweist.

Für die klassischen Anwendungen, d. h. mit Lichtquellen im nahen UV-Bereich, sind dazu insbesondere Bindemittel auf der Basis von Novolak-Kondensationsharzen, die in der Regel in Kombination mit Naphthochinondiaziden als photoaktive Komponenten eingesetzt worden sind, geeignet. Solche Phenol-Formaldehyd-Kondensate sind mehrfach beschrieben worden und können als phenolische Komponente Phenol, die drei stellungsisomeren Kresole oder andere Alkylphenole, z. B. Xylenole, als Komponenten enthalten. Neben Formaldehyd können auch andere Aldehyde zur Herstellung des Polymeren herangezogen werden. Ebenso gut lassen sich die nachstehend beschriebenen, Hydroxylgruppen enthaltenden Polymeren für Bestrahlungen mit nahem UV-Licht verwenden.

Für Anwendungen im UV2-Bereich sind jedoch andere polymere Materialien erforderlich. Novolake, die in der Regel in Kombination mit Naphthochinondiaziden als photoaktive Komponenten eingesetzt werden, sind dafür ungeeignet. Sie können aber in Mischung mit anderen, als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 50 Gew.-%, insbesondere bis zu 20 Gew.-%, eines Novolak-Kondensationsharzes enthalten.

Als Bindemittel geeignet sind Homo- oder Copolymere des p-Hydroxystyrols sowie seiner Alkylderivate, z. B. des 3-Methyl-4-hydroxystyrols, des 3,5-Dimethyl-4-hydroxystyrols oder des 2,3-Dimethyl-4-hydroxystyrols sowie Homo- oder Copolymere anderer Polyvinylphenole, z. B. des 3-Hydroxystyrols, oder des 4-Methyl-3-hydroxystyrols oder die Ester der (Meth)acrylsäure mit Phenolen, z. B. Brenzkatechin, Resorcin, Hydrochinon, Pyrogallol oder Aminophenole, sowie die entsprechenden Amide mit aromatischen Aminen. Als Comonomere können polymerisierbare Verbindungen wie Styrol, Methylmethacrylat, Methylacrylat oder ähnliche eingesetzt werden.

Gemische mit erhöhter Plasmabeständigkeit werden dann erhalten, wenn zur Herstellung von Copolymeren des obigen Typs Silicium enthaltende Vinylmonomere, z. B. Vinyl- oder Allyl-trimethylsilan, verwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleinimids verwenden. Auch diese Bindemittel zeigen hohe Transparenz im beschriebenen Wellenlängenbereich. Als Comonomere werden auch hier bevorzugt Styrol, substituierte Styrole, Vinylphenole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierunter zählen beispielsweise Maleinsäureanhydrid und Maleinsäurehalbester.

Die genannten Bindemittel können dann untereinander gemischt werden, wenn sich dadurch die optische Qualität des strahlungsempfindlichen Gemisches nicht verschlechtert. Bindemittelgemische sind jedoch nicht bevorzugt.

Die Menge des Bindemittels beträgt im allgemeinen 30 bis 95 Gew.-%, insbesondere 40 bis 90 Gew.-%, vorzugsweise 50 bis 85 Gew.-%, bezogen auf das Gesamtgewicht der festen Anteile der strahlungsempfindlichen Mischung.

Die Extinktion des Bindemittels bzw. der Kombination von Bindemitteln für Strahlung der Wellenlänge von etwa 220 bis 500 nm sollte weniger als 0,5, bevorzugt weniger als 0,3 $\mu m^{-1}$, betragen.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Erfüllung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Hierfür besonders geeignet sind Ethylenglykol und Propylenglykol sowie die davon abgeleiteten Mono- und Dialkylether, besonders die Mono- und Dimethylether sowie die Mono- und Diethylether, Ester abge-

leitet aus aliphatischen ($C_1$-$C_6$)Carbonsäuren und entweder ($C_1$-$C_8$)Alkanolen oder ($C_1$-$C_8$)Alkandiolen oder ($C_1$-$C_6$)Alkoxy-($C_1$-$C_8$)alkanolen, beispielsweise Ethylacetat, Hydroxyethylacetat, Alkoxyethylacetat, n-Butylacetat, Propylenglykolmonoalkyletheracetat, insbesondere Propylenglykolmethyletheracetat und Amylacetat, Ether, wie Tetrahydrofuran und Dioxan, Ketone, wie Methylethylketon, Methylisobutylketon, Cyclopentanon und Cyclohexanon, N,N-Dialkyl-carbonsäureamide, wie N,N-Dimethylformamid und N,N-Dimethylacetamid, aber auch Hexamethylphosphorsäuretriamid, 1-Methyl-pyrrolidin-2-on und Butyrolacton, sowie beliebige Mischungen davon. Besonders bevorzugt von diesen sind die Glykolether, aliphatischen Ester und Ketone.

Letztendlich hängt die Wahl des Lösemittels bzw. Lösemittelgemisches ab von dem angewandten Beschichtungsverfahren, der gewünschten Schichtstärke und den Trocknungsbedingungen. Ebenso müssen die Lösemittel chemisch neutral sein, d. h. sie dürfen nicht mit den übrigen Schichtkomponenten irreversibel reagieren.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht aus dem erfindungsgemäßen strahlungsempfindlichen Gemisch besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Speziell sind Siliciumsubstrate zu nennen, die auch thermisch oxidiert und/oder mit Aluminium beschichtet, aber auch dotiert sein können. Daneben sind alle anderen in der Halbleitertechnologie üblichen Substrate möglich, wie Siliciumnitrid, Galliumarsenid und Indiumphosphid. Weiterhin kommen die aus der Flüssigkristalldisplay-Herstellung bekannten Substrate in Frage, wie Glas oder Indium-Zinnoxid, ferner Metallplatten und -folien - beispielsweise aus Aluminium, Kupfer, Zink-, Bimetall- und Trimetallfolien, aber auch elektrisch nichtleitende Folien, die mit Metallen bedampft sind, und Papier. Diese Substrate können thermisch vorbehandelt, oberflächlich aufgerauht, angeätzt oder zur Verbesserung erwünschter Eigenschaften, z. B. zur Erhöhung der Hydrophilie, mit Chemikalien vorbehandelt sein.

Um der strahlungsempfindlichen Schicht einen besseren Zusammenhalt und/oder eine bessere Haftung auf der Substratoberfläche zu verleihen, kann in ihr ein Haftvermittler enthalten sein. Der gleiche Effekt läßt sich mit einer haftvermittelnden Zwischenschicht erreichen. Bei Silicium- bzw. Siliciumdioxid-Substraten kommen hierfür Haftvermittler vom Aminosilan-Typ, wie z. B. 3-Aminopropyltriethoxysilan oder Hexamethyldisilazan, in Frage.

Geeignete Träger für die Herstellung von photomechanischen Aufzeichnungsschichten, wie Druckformen für den Hochdruck, Flachdruck, Siebdruck und Flexodruck sind besonders Aluminiumplatten, die gegebenenfalls vorher anodisch oxidiert, gekörnt und/oder silikatisiert werden, daneben Zink- und Stahlplatten, die gegebenenfalls verchromt werden, sowie Kunststoffolien und Papier.

Das erfindungsgemäße Aufzeichnungsmaterial wird mit aktinischer Strahlung bildmäßig belichtet. Als Strahlungsquellen eignen sich besonders Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Als aktinische Strahlung soll im Rahmen dieser Beschreibung jede Strahlung verstanden werden, deren Energie mindestens der des kurzwelligen sichtbaren Lichts entspricht. Geeignet ist daher insbesondere langwellige und kürzerwellige UV-Strahlung, wie sie beispielsweise von Excimer-Lasern ausgesandt wird.

Die Stärke der lichtempfindlichen Schicht hängt vom Verwendungszweck ab. Sie beträgt im allgemeinen zwischen 0,1 und 100 $\mu$m, bevorzugt zwischen 1 und 10 $\mu$m.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann durch Erhitzen der Schicht auf Temperaturen bis zu 150 °C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien geeigneten Materialien Anwendung finden. Anschließend wird die Schicht bildmäßig bestrahlt. Danach behandelt man die Schicht mit einer Entwicklerlösung, die die bestrahlten Bereiche der Schicht löst und entfernt, so daß ein Abbild der bei der bildmäßigen Bestrahlung verwendeten Vorlage auf der Substratoberfläche verbleibt.

Als Entwickler eignen sich besonders wäßrige Lösungen, die Silikate, Metasilikate, Hydroxide, Hydrogen- und Dihydrogenphosphate, Carbonate oder Hydrogencarbonate von Alkalimetall-, Erdalkalimetall- und/oder Ammoniumionen enthalten, aber auch Ammoniak und dergleichen. Metallionenfreie Entwickler werden in der US-A 4 729 941, der EP-A 0 062 733, der US-A 4 628 023, der US-A 4 141 733, der EP-A 0 097 282 und der EP-A 0 023 758 beschrieben. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung. Metallionenfreie Entwickler werden bevorzugt verwendet. Den Entwicklern können geringe Mengen eines Netzmittels zugesetzt sein, um die Ablösung der löslichen Bereiche der Schicht zu erleichtern.

Die entwickelten Schichtstrukturen können nachgehärtet werden. Dies geschieht im allgemeinen durch Erhitzen auf einer hot plate bis zu einer Temperatur unterhalb der Fließtemperatur und anschließendes ganzflächiges Belichten mit dem UV-Licht einer Xenon-Quecksilber-Dampflampe (Bereich von 200 bis 250 nm). Durch die Nachhärtung werden die Bildstrukturen vernetzt, so daß sie im allgemeinen eine Fließbeständigkeit bis zu Temperaturen von über 200 °C aufweisen. Die Nachhärtung kann auch ohne Temperaturerhöhung allein durch Bestrahlen mit energiereichem UV-Licht in hoher Dosis erfolgen.

Verwendung findet das erfindungsgemäße strahlungsempfindliche Gemisch bei der Herstellung von integrierten Schaltungen oder von einzelnen elektrischen Bausteinen mit lithographischen Prozessen, da sie eine hohe Lichtempfindlichkeit aufweisen, besonders bei Bestrahlung mit Licht einer Wellenlänge zwischen 190 bis 300 nm. Da die Gemische bei Belichtung sehr gut ausbleichen, lassen sich feinere Strukturen erzielen als dies mit den bekannten Gemischen möglich ist. Die entwickelte Resistschicht dient dabei als Maske für die folgenden Prozeßschritte. Solche Schritte sind z. B. das Ätzen des Schichtträgers, das Implantieren von Ionen in den Schichtträger oder das Abscheiden von Metallen oder anderen Materialien auf dem Schichtträger.

Die nachstehend beschriebenen Beispiele illustrieren die Erfindung, sollen aber keine Einschränkung bewirken. Prozentangaben sind immer Gewichtsprozente, Schmelzpunkte sind, sofern nicht anders angegeben, unkorrigiert. Gt steht im Folgenden für Gewichtsteile.

Beispiel 1

1. Stufe: Herstellung von 4-tert.-Butoxycarbonyloxy-benzaldehyd-dimethylacetal:

2 g (9 mmol) 4-(tert.-Butoxycarbonyloxy)-benzaldehyd wurden bei Raumtemperatur in 10 ml Methanol und 1,2 ml Orthoameisensäuretrimethylester gelöst. Um eine vollständige Lösung zu erhalten, wurden 1,5 ml Toluol zugefügt. Dann erfolgte die Zugabe von 3,75 mg $RhCl_3[CH_3C(CH_2PPh_2)_3]$ als Katalysator. Anschließend wurde die Mischung über Nacht bei Raumtemperatur gerührt. Die Lösemittel wurden durch Wasserdampfdestillation abgetrennt und der ölige Rückstand in wenig Toluol aufgenommen. Der Rhodiumkatalysator wurde über eine G-4-Fritte abfiltriert. Nach dem Abziehen des Toluols unter vermindertem Druck wurde das Produkt im Ölpumpenvakuum getrocknet.

Ausbeute 2,3 g (95% d.Th.), schwach gelbes Öl, H-NMR ($CDCl_3$): Acetalsignal bei 5,38 ppm, BOC-Signal bei 1,56 ppm, im IR-Spektrum CO-Signal bei 1760 $cm^{-1}$.

2. Stufe: Herstellung von 4-tert.-Butoxycarbonyloxy-benzaldehyd-bis-(2-phenoxyethyl)-acetal:

Eine Mischung aus 2 g (7,45 mmol) 4-(tert.-Butoxycarbonyloxy)-benzaldehyd-dimethylacetal, 2,06 g (14,9 mmol) 2-Phenoxyethanol und 40 mg Kaliumhydrogensulfat wurde in 30 ml getrocknetem Toluol am Rückflußkühler zum Sieden erhitzt. Nach 1 Stunde Erhitzen wurde das entstandende Methanol langsam abdestilliert, während erneut Toluol zur Mischung gegeben wurde. Nach vollständiger Entfernung des Methanols wurde die Mischung abgekühlt, der Kaliumhydrogensulfat-Katalysator abfiltriert und das Toluol abgezogen. Der ölige Rückstand wurde in Dichlormethan gelöst und mit Wasser gewaschen. Die organische Phase wurde über Magnesiumsulfat getrocknet. Nach dem Abziehen des Lösemittels unter vermindertem Druck wurde das Produkt im Ölpumpenvakuum getrocknet.

Ausbeute 3,26 g (91% d.Th.), schwach gelbes Öl, das nach 2 Tagen auskristallisiert ist (Smp. 39 bis 40 °C). H-NMR ($CDCl_3$): Acetalsignal bei 5,77 ppm, BOC-Signal bei 1,57 ppm, im IR-Spektrum CO-Signal bei 1760 $cm^{-1}$. Das Massenspektrum zeigt die Molmasse M = 480.

Beispiel 2

1. Stufe: Herstellung von 3-tert.-Butoxycarbonyloxy-benzaldehyd-dimethylacetal:

2 g (9 mmol) 3-tert.-Butoxycarbonyloxy-benzaldehyd wurden bei Raumtemperatur in 10 ml Methanol und 1,2 ml Orthoameisensäuretrimethylester

gelöst. Um vollständige Lösung zu erreichen, wurden 1,5 ml Toluol zugefügt. Dann erfolgte die Zugabe von 3,75 mg $RhCl_3[CH_3C(CH_2PPh_2)_3]$ als Katalysator. Anschließend wurde die Mischung über Nacht bei Raumtemperatur gerührt. Die Lösemittel wurden durch Wasserdampfdestillation abgetrennt und der ölige Rückstand in wenig Toluol aufgenommen. Der Rhodiumkatalysator wurde über eine G-4-Fritte abfiltriert. Nach dem Abziehen des Toluols unter vermindertem Druck wurde das Produkt im Ölpumpenvakuum getrocknet.

Ausbeute 2,1 g (85% d.Th.), schwach gelbes Öl, H-NMR ($CDCl_3$): Acetalsignal bei 5,35 ppm, BOC-Signal bei 1,55 ppm, im IR-Spektrum CO-Signal bei 1760 $cm^{-1}$.

2. Stufe: Herstellung von 3-tert.-Butoxycarbonyloxy-benzaldehyd-bis-(2-phenylethyl)-acetal:

1 g (3,73 mmol) 3-tert.-Butoxycarbonyloxy-benzaldehyd-dimethylacetal wurden in 40 ml getrocknetem Toluol gelöst. Nach Zugabe von 0,91 g (7,46 mmol) 2-Phenylethanol und 18 mg Kaliumhydrogensulfat wurde die resultierende Mischung 2 h unter Rückfluß gerührt. Anschließend wurde das entstandende Methanol langsam abdestilliert, währende erneut Toluol zur Mischung gegeben wurde. Nach vollständiger Entfernung des Methanols wurde die Mischung abgekühlt und das Kaliumhydrogensulfat abfiltriert. Das Filtrat wurde am Rotationsverdampfer eingeengt. Dabei wurde ein gelbliches, viskoses Öl erhalten, das im Ölpumpenvakuum getrocknet wurde.

Ausbeute 1,59 g (95% d.Th.). H-NMR ($CDCl_3$): Acetalsignal bei 5,50 ppm, BOC-Signal bei 1,57 ppm, im IR-Spektrum CO-Signal bei 1760 $cm^{-1}$.

Beispiel 3

1. Stufe: Herstellung von 3-Methoxy-4-tert.-butoxycarbonyloxy-benzaldehyd-diethylacetal:

1,52 g (6 mmol) 3-Methoxy-4-tert.-butoxycarbonyloxy-benzaldehyd wurden in 18 ml Ethanol und 2 ml Orthoameisensäuretriethlester gelöst. Zur vollständigen Lösung des 3-Methoxy-4-tert.-butoxycarbonyloxy-benzaldehyds wurden weitere 2 ml Toluol zugefügt. Dann erfolgte die Zugabe von 4,2 mg $RhCl_3[CH_3C(CH_2PPh_2)_3]$ als Katalysator. Anschließend wurde die Reaktionslösung so lange bei Raumtemperatur gerührt bis im Dünnschichtchromatogramm kein Ausgangsmaterial mehr feststellbar war. Nach dem Abziehen der Lösemittel wurde der olige Rückstand in wenig Toluol gelöst und über eine G-4-Fritte filtriert. Die erhaltene Lösung wurde am Rotationsverdampfer eingeengt und im Ölpumpenvakuum getrocknet.

Ausbeute 1,72 g (88% d.Th.), schwach gelbes Öl, H-NMR ($CDCl_3$): Acetalsignal bei 5,44 ppm, BOC-Signal bei 1,55 ppm, $CH_3O$-Signal bei 3,73 ppm, im IR-Spektrum CO-Signal bei 1760 $cm^{-1}$.

2. Stufe: Herstellung von 3-Methoxy-4-tert.-butoxycarbonyloxy-benzaldehyd-di(phenylethyl)acetal:

1,1 g (3,36 mmol) 3-Methoxy-4-tert.-butoxycarbonyloxy-benzaldehyd-di(ethyl)acetal wurden in 40 ml getrocknetem Toluol gelöst. Nach Zugabe von 0,82 g (6,71 mmol) Phenylethanol und 16 mg Kaliumhydrogensulfat wurde die resultierende Mischung 2 h unter Rückfluß gerührt. Anschließend wurde das entstandene Ethanol langsam abdestilliert, während erneut Toluol zur Mischung gegeben wurde. Nach vollständiger Entfernung des Ethanols wurde die Mischung abgekühlt und das Kaliumhydrogensulfat abfiltriert. Das Filtrat wurde am Rotationsverdampfer eingeengt. Dabei wurde ein gelbliches, viskoses Öl erhalten, das an der Ölpumpe getrocknet wurde.

Ausbeute 1,56 g (97% d.Th.), H-NMR ($CDCl_3$): Acetalsignal bei 5,45 ppm, BOC-Signal bei 1,55 ppm, im IR-Spektrum CO-Signal bei 1760 $cm^{-1}$.

Beispiel 4

1. Stufe: Herstellung von 4-tert.-Butoxycarbonyloxy-benzylalkohol:

10 g (137,5 mmol) $K_2CO_3$ wurden in 150 ml $CH_2Cl_2$ suspendiert und 6,2 g (50 mmol) 4-Hydroxybenzylalkohol zugegegeben. Der Alkohol löste sich nicht vollständig. Das Reaktionsgemisch wurde auf 0 °C abgekühlt. Bei dieser Temperatur wurden 660 mg (2,5 mmol) 18-Krone-6-ether in 10,7 ml = 10,9 g (50 mmol) Di-tert.-butyldicarbonat zugetropft. Es wurde einige Zeit bei 0 °C und über Nacht bei Raumtemperatur gerührt. Danach war kein Ausgangsmaterial mehr feststellbar, wie ein Dünnschichtchromatogramm ($SiO_2$, Petrolether:Essigester 1:1) zeigte. Anschließend wurde das Kaliumcarbonat abfiltriert. Das Reaktionsgemisch wurde mit Methylenchlorid verdünnt, dreimal mit gesättigter Natriumchloridlösung gewaschen und dann über Magnesiumsulfat getrocknet. Das Magnesiumsulfat wurde abfiltriert, das Filtrat eingeengt und anschließend über eine $SiO_2$-Säule mit Petrolether:Essigester (1:1) chromatographiert. Man erhielt als Produkt 8 g (71%d.Th.) eines farblosen Öls.

2. Stufe: Herstellung von 4-tert.-Butoxycarbonyloxy-benzaldehyd-bis-(4-tert.-butoxycarbonyloxy-benzyl)-acetal:

Eine Mischung von 1 g (3,73 mmol) 4-tert.-Butoxycarbonyloxy-benzaldehyd-dimethylacetal, hergestellt entsprechend Stufe 1 des Beispiels 1, 1,67 g (7,46 mmol) 4-tert.-Butoxycarbonyloxy-benzylalkohol und 18 mg Kaliumhydrogensulfat wurde in 40 ml getrocknetem Toluol 1 h unter Rückfluß erhitzt. Danach wurde das entstandende Methanol unter Zugabe von Toluol langsam abdestilliert. Nach vollständiger Entfernung des Methanols wurde die Mischung abgekühlt, der Katalysator abfiltriert und das Toluol abgezogen. Der ölige Rückstand wurde im Ölpumpenvakuum getrocknet.

Ausbeute 2,33 g (96% d.Th.), schwach gelbes Wachs, H-NMR ($CDCl_3$): Acetalsignal bei 5,71 ppm, BOC-Signal bei 1,55 ppm, $CH_2O$-Signal bei 4,54 ppm, im IR-Spektrum CO-Signal bei 1760 $cm^{-1}$.

Beispiel 5

1. Stufe: Herstellung von 2,3-Bis-tert.-butoxycarbonyloxy-benzaldehyd-dimethylacetal:

3 g (9 mmol) 2,3-Bis-tert.-butoxycarbonyloxy-benzaldehyd wurden bei Raumtemperatur in 10 ml Methanol und 1,2 ml Orthoameisensäuretrimethylester gelöst. Um das Benzaldehydderivat vollständig zu lösen, wurden 1,5 ml Toluol zugegeben. Dann erfolgte die Zugabe von 3,75 mg $RhCl_3[CH_3C(CH_2PPh_2)_3]$ als Katalysator. Anschließend wurde die Mischung über Nacht bei Raumtemperatur gerührt. Die Lösemittel wurden durch Wasserdampfdestillation abgetrennt und der ölige Rückstand in wenig Toluol aufgenommen. Der ungelöste Rhodiumkatalysator wurde über eine G-4-Fritte abfiltriert. Nach dem Abziehen des Toluols unter vermindertem Druck wurde das Produkt im Ölpumpenvakuum getrocknet.

Ausbeute 2,9 g (75% d.Th.), schwach gelbes Öl, H-NMR ($CDCl_3$): Acetalsignal bei 5,30 ppm, BOC-Signal bei 1,55 ppm, im IR-Spektrum CO-Signal bei 1760 $cm^{-1}$.

2. Stufe: Herstellung von 2,3-Bis-tert.-butoxycarbonyloxy-benzaldehyd-bis-(2-phenylethyl)-acetal:

1,43 g (3,73 mmol) 2,3-Bis-tert.-butoxycarbonyloxy-benzaldehyd-dimethylacetal wurden in 40 ml getrocknetem Toluol gelöst. Nach Zugabe von 0,91 g (7,46 mmol) 2-Phenylethanol und 18 mg Kaliumhydrogensulfat wurde die resultierende Mischung 1 h unter Rückfluß gerührt. Anschließend wurde das entstandende Methanol langsam abdestilliert, während erneut Toluol zur Mischung gegeben wurde. Nach vollständiger Entfernung des Methanols wurde die Mischung abgekühlt und das Kaliumhydrogensulfat abfiltriert. Das Filtrat wurde am Rotationsverdampfer eingeengt. Dabei wurde ein gelbliches, viskoses Öl erhalten, das im Ölpumpenvakuum getrocknet wurde.

Ausbeute 1,66 g (79% d.Th.), H-NMR ($CDCl_3$): Acetalsignal bei 5,48 ppm, BOC-Signal bei 1,54 ppm, im IR-Spektrum CO-Signal bei 1760 $cm^{-1}$.

Beispiele 6 bis 44

Die nachstehenden Verbindungsbeispiele wurden analog der in Beispiel 1 und/oder Beispiel 2 angegebenen Vorschrift hergestellt, wobei im Einzelfall lediglich geringfügige Änderungen der Reaktionsdauer und der Aufarbeitung durchgeführt wurden. Alle Verbindungen konnten eindeutig durch Elementaranalyse, H-NMR und IR-Spektrum identifiziert werden.

6. 4-tert.-Butoxycarbonyloxy-3-nitro-benzaldehyd-diphenethylacetal
7. 4-tert.-Butoxycarbonyloxy-3,5-dimethyl-benzaldehyd-dimethylacetal
8. 4-tert.-Butoxycarbonyloxy-benzaldehyd-dipropylacetal
9. 4-tert.-Butoxycarbonyloxy-benzaldehyd-diisopropylacetal
10. 4-tert.-Butoxycarbonyloxy-benzaldehyd-dibutylacetal
11. 4-tert.-Butoxycarbonyloxy-3-methyl-benzaldehyd-bis-(2-cyclohexyl-ethyl-acetal)
12. 4-tert.-Butoxycarbonyloxy-benzaldehyd-bis-(2-ethoxyethyl-acetal)
13. 3-tert.-Butoxycarbonyloxy-4-nitro-benzaldehyd-dimethylacetal
14. 2-tert.-Butoxycarbonyloxy-naphthalin-1-carbaldehyd-diethylacetal
15. 2-tert.-Butoxycarbonyloxy-benzaldehyd-diethylacetal
16. 4-tert.-Butoxycarbonyloxy-3-methyl-benzaldehyd-dihexylacetal
17. 4-tert.-Butoxycarbonyloxy-2-methyl-benzaldehyd-dipropylacetal
18. 4-tert.-Butoxycarbonyloxy-benzaldehyd-bis-[3-(N'-propyl-ureido)-propyl-acetal]
19. 4-tert.-Butoxycarbonyloxy-2-methyl-benzaldehyd-diallylacetal
20. 3-tert.-Butoxycarbonyloxy-4-methoxy-benzaldehyd-bis-(3-phenoxy-propyl-acetal)
21. 3-tert.-Butoxycarbonyloxy-4-methyl-benzaldehyd-diphenethylacetal
22. 3-tert.-Butoxycarbonyloxy-benzaldehyd-bis-(3-phenylcarbamoyloxy-propyl-acetal)
23. 4-tert.-Butoxycarbonyloxy-3-methyl-benzaldehyd-bis-(2-phenoxy-ethyl-acetal)
24. 4-tert.-Butoxycarbonyloxy-2-methyl-benzaldehyd-bis-(2-benzolsulfonyloxy-ethyl-acetal)
25. 4-tert.-Butoxycarbonyloxy-3-methyl-benzaldehyd-bis-(2-benzolsulfonylamino-ethyl-acetal)
26. 4-tert.-Butoxycarbonyloxy-benzaldehyd-bis-(3,6,9-tetraoxa-undecyl-acetal)

27. 4-tert.-Butoxycarbonyloxy-benzaldehyd-bis-[2-(4-chlor-phenoxy)-ethyl]-acetal
28. 4-tert.-Butoxycarbonyloxy-3-methyl-benzaldehyd-bis-[3-(4-cyano-phenyl)-propyl-acetal]
29. 3-tert.-Butoxycarbonyloxy-benzaldehyd-bis-[3-(4-brom-phenoxy)-propyl-acetal]
30. 4-tert.-Butoxycarbonyloxy-3-methyl-benzaldehyd-bis-(2-diphenyl-4-yloxy-ethyl-acetal)
31. 4-tert.-Butoxycarbonyloxy-benzaldehyd-bis-(2-benzoylamino-ethyl-acetal)
32. 3,4-Bis-tert.-butoxycarbonyloxy-benzaldehyd-bis-(2-benzoyloxy-ethyl-acetal)
33. 4-tert.-Butoxycarbonyloxy-benzaldehyd-bis-(2-ethansulfonyl-ethyl-acetal)
34. 4-tert.-Butoxycarbonyloxy-benzaldehyd-bis-[2-(4-methoxy-phenoxy)-ethyl-acetal]
35. 4-tert.-Butoxycarbonyloxy-3-(2-methoxy-ethoxy)-benzaldehyd-bis-(3-butylcarbamoyloxy-propyl-acetal)
36. 3-tert.-Butoxycarbonyloxy-4-phenyl-benzaldehyd-bis-(3-phenoxy-propyl-acetal)
37. 3,4,5-Tris-tert.-butoxycarbonyloxy-benzaldehyd-bis-[2-(N-ethoxycarbonylmethyl-carbamoyloxy)-ethyl-acetal]
38. 4-tert.-Butoxycarbonyloxy-benzaldehyd-bis-[2-(benzoyl-ethyl-amino)-ethyl-acetal]
39. 4-tert.-Butoxycarbonyloxy-benzaldehyd-diprop-2-inylacetal
40. 4-tert.-Butoxycarbonyloxy-benzophenon-bis-(2-phenoxy-ethyl-acetal)
41. 4,4'-Bis-tert.-butoxycarbonyloxy-benzophenon-bis-(3-phenoxy-propyl-acetal)
42. 4-tert.-Butoxycarbonyloxy-orthobenzoesäure-tris-(2-ethoxy-ethyl-ester)
43. 4-tert.-Butoxycarbonyloxy-benzaldehyd-bis-(decahydronaphthalin-1-yl-acetal)
44. 4-tert.-Butoxycarbonyloxy-benzaldehyd-bis-(4-but-2-inyloxy-cyclohexyl-acetal)

Anwendungsbeispiel 1:

Es wurde eine Beschichtungslösung hergestellt aus
7,5 Gt eines Kresol-Formaldehyd-Novolaks mit einem Erweichungsbereich von 105 bis 120 °C,
2,0 Gt 4-tert.-Butoxycarbonyloxy-benzaldehyd-bis-(2-phenoxyethyl)-acetal,
0,4 Gt 6(4-Chlorstyryl)-4-methyl-1-trifluormethansulfonyloxy-2-pyridon und
42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.200 Umdrehungen je Minute aufgeschleudert. Nach 1 min Trocknen bei 100 °C auf der hot plate wurde eine Schichtdicke von 1,1 µm erhalten.

Das Aufzeichnungsmaterial wurde bildmäßig unter einer Vorlage mit der Strahlung einer Xenon-Quecksilberdampflampe bei 365 nm einer Energie von 55 mJ/cm$^2$ belichtet. Um die Spaltung des Lösungsinhibitors zu vervollständigen, wurde das Material 1 min auf 100 °C erwärmt.

Entwickelt wurde das Aufzeichnungsmaterial mit einem rein wäßrigen Entwickler, der 2,38 Gew.-% Tetramethylammoniumhydroxid enthielt.

Nach einer Entwicklungsdauer von 120 s erhielt man ein fehlerfreies Abbild der Maske mit steilen Resistflanken, wobei auch Strukturen < 0,55 µm detailgetreu aufgelöst waren. Eine Untersuchung der Flanken der Resistprofile mit Rasterelektronenmikroskopie belegte, daß diese praktisch senkrecht zur Substratoberfläche ausgerichtet waren. Der Kontrast betrug 4,6.

Der Kontrast eines Positivresists, $c_p$, ist definiert als

$$c_p = 1 / (\log D_p - \log D_p^o) = [\log(D_p/D_p^o)]^{-1}$$

wobei $D_p^o$ die Einstrahlungsdosis ist, bei welcher der Entwickler beginnt, den belichteten Film anzugreifen, und $D_p$ den Resistaufpunkt (= Resistempfindlichkeit) darstellt. Eine genaue Beschreibung dieser Größe enthält der Aufsatz von L.F. Thompson und M.J. Bowden "Resist Processing" (Introduction to Microlithography, Theory, Materials and Processing, Herausgeber C.G. Willson, L.F. Thompson, und M.J. Bowden, ACS Symp. Ser., 219, 164 ff (1983), American Chemical Society, Washington).

Anwendungsbeispiel 2:

Es wurde eine Beschichtungslösung hergestellt aus
7,5 Gt eines Copolymeren aus 3,5-Dimethyl-4-hydroxystyrol/4-Hydroxystyrol (Molverhältnis 30:70) mit einem mittleren Molekulargewicht von 25.000,
2,0 Gt 2,3-Bis-tert.-butoxycarbonyloxy-benzaldehyd-diphenethylacetal und
0,4 Gt Triphenylsulfonium-trifluormethansulfonat in
42 Gt Propylenglykol-monomethylether-acetat.

Die Lösung wurde durch ein Filter mit einem Porendurchmesser von 0,2 µm filtriert und auf einen mit einem Haftvermittler (Hexamethyldisilazan) behandelten Wafer bei 3.300 Umdrehungen aufgeschleudert. Nach 1 min Trocknen bei 100 °C wurde eine Schichtdicke von 1,08 µm erhalten.

Das Aufzeichnungsmaterial wurde bildmäßig unter einer Vorlage mit der Strahlung einer Xenon-Quecksilberdampflampe bei 240 bis 260 nm mit einer Energie von 35 mJ/cm$^2$ belichtet, 75 s auf 100 °C erwärmt und anschließend mit dem in Anwendungsbeispiel 1 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,4 µm detailgetreu aufgelöst waren.

Anwendungsbeispiel 3:

Es wurde eine Beschichtungslösung hergestellt aus

7,5 Gt eines Copolymeren aus Styrol und Maleimid (Molverhältnis 1:1) mit einem Erweichungsbereich von 165 bis 180 °C,

2,0 Gt 3-tert.-Butoxycarbonyloxy-benzaldehyd-diphenethylacetal und

0,3 Gt Triphenylsulfonium-trifluormethansulfonat in

42 Gt Cyclohexanon.

Die Lösung wurde durch ein Filter mit einem Porendurchmesser von 0,2 µm filtriert und auf einen mit einem Haftvermittler (Hexamethyldisilazan) behandelten Wafer bei 3.400 Umdrehungen aufgeschleudert. Nach 1 min Trocknen bei 100 °C wurde eine Schichtdicke von 0,98 µm erhalten.

Das Aufzeichnungsmaterial wurde bildmäßig unter einer Vorlage mit der Strahlung einer Xenon-Quecksilberdampflampe bei 240 bis 260 nm mit einer Energie von 49 mJ/cm$^2$ belichtet.

Entwickelt wurde das Aufzeichnungsmaterial mit einer 0,02 n wäßrigen Tetramethylammoniumhydroxid-Lösung, wobei die belichteten Bereiche innerhalb von 60 s rückstandsfrei abgelöst wurden.

Man erhielt wiederum ein fehlerfreies Abbild der Maske mit steilen Resistflanken. Der Dunkelabtrag war < 20 nm; auch Strukturen < 0,4 µm waren detailgetreu aufgelöst.

Anwendungsbeispiel 4:

Es wurde eine Beschichtungslösung hergestellt aus

7,5 Gt eines Homopolymeren aus 3-Methyl-4-hydroxystyrol mit einem mittleren Molekulargewicht von 25.000,

2,0 Gt 4-tert.-Butoxycarbonyloxy-3-methoxy-benzaldehyd-diphenethylacetal und

0,4 Gt 4-Methyl-6-phenyl-1-trifluormethansulfonyloxy-2-pyridon in

42 Gt Propylenglykol-monomethylether-acetat.

Die Lösung wurde durch ein Filter mit einem Porendurchmesser von 0,2 µm filtriert und in zwei gleiche Teile getrennt. Ein Teil wurde auf einen mit einem Haftvermittler (Hexamethyldisilazan) behandelten Wafer bei 3.300 Umdrehungen aufgeschleudert. Nach 1 min Trocknen bei 100 °C wurde eine Schichtdicke von 1,04 µm erhalten.

Das Aufzeichnungsmaterial wurde bildmäßig unter einer Vorlage mit der Strahlung einer Xenon-Quecksilberdampflampe bei 240 bis 260 nm mit einer Energie von 62 mJ/cm$^2$ belichtet, 75 s auf 100 °C erwärmt und anschließend mit dem in Anwendungsbeispiel 1 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,4 µm detailgetreu aufgelöst waren.

Der zweite Teil wurde nach 20 Wochen Lagerung im Kühlschrank der gleichen Prozedur unterzogen. Es wurden identische Ergebnisse erhalten. Daraus geht hervor, daß das Gemisch in Lösung außerordentlich gute Stabilität aufweist.

Anwendungsbeispiel 5:

Das in Anwendungsbeispiel 1 beschriebene Material wurde bildmäßig mit Strahlung einer Wellenlänge von 436 nm belichtet und wie dort angegeben verarbeitet. Es wurde eine korrekte Vorlagenwiedergabe bis herab zu 0,55 µm beobachtet.

## Patentansprüche

**1.** Verbindungen mit der allgemeinen Formel I

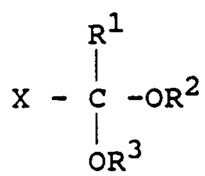

(I)

worin

X

ein Phenyl-, [1]Naphthyl- oder [2]-Naphthylrest ist, der jeweils mit mindestens einer tert.-Butoxycarbonyloxygruppe substituiert ist und gegebenfalls einen oder mehr als einen zusätzlichen Substituenten aufweist,

$R^1$

für ein Wasserstoffatom, einen $(C_1$-$C_6)$Alkyl-, einen $(C_6$-$C_{10})$Arylrest oder einen der Reste X steht und

$R^2$ und $R^3$

gleich oder verschieden sind und einen $(C_1$-$C_{12})$Alkylrest, in dem gegebenenfalls bis zu drei Methylengruppen durch Brückenglieder, die mindestens ein Heteroatom aufweisen, wie -O-, -S-, -$NR^4$-, -CO-, -CO-O-, -CO-NH-, -O-CO-NH-, -NH-CO-NH-, -CO-NH-CO-, -$SO_2$-, -$SO_2$-O-, oder -$SO_2$-NH-, ersetzt sind, einen $(C_3$-$C_{12})$Alkenyl-, $(C_3$-$C_{12})$-Alkinyl, $(C_4$-$C_{12})$Cycloalkyl-, $(C_4$-$C_{12})$-Cycloalkenyl- oder $(C_8$-$C_{16})$Aralkylrest, in dem gegebenenfalls bis zu drei Methylengruppen des aliphatischen Teils durch Brük-

kenglieder der oben genannten Art ersetzt sind und der im aromatischen Teil durch Fluor-, Chlor- oder Bromatome, durch ($C_1$-$C_4$)Alkyl-, ($C_1$-$C_4$)Alkoxy-, Nitro-, Cyano- oder tert.-Butoxycarbonyloxygruppen substituiert sein kann, bedeuten, wobei

$R^4$

für einen Acyl-, insbesondere einen ($C_1$-$C_6$)-Alkanoylrest, steht.

**2.** Verbindungen gemäß Anspruch 1, dadurch gekennzeichnet, daß die gegebenenfalls zusätzlich in den Resten X vorkommenden Substituenten Fluor-, Chlor-, Brom- oder Jodatome, Nitro-, Cyano- oder Carboxygruppen, ($C_1$-$C_9$)-, besonders bevorzugt ($C_1$-$C_6$)Alkylreste, in denen gegebenenfalls bis zu drei, besonders bevorzugt bis zu zwei, Methylengruppen durch Brückenglieder der im Anspruch 1 genannten Art ersetzt sind, Phenylreste, die wiederum substituiert sein können, insbesondere mit tert.-Butoxycarbonylgruppen, ($C_1$-$C_4$)-Alkylresten, ($C_1$-$C_4$)Alkoxyresten und/oder Halogen-atomen, sowie ($C_8$-$C_{12}$)-, besonders bevorzugt ($C_8$-$C_{10}$)Aralkylreste, in denen gegebenenfalls bis zu zwei Methylengruppen durch Brückenglieder der oben genannten Art ersetzt sind, ($C_6$-$C_{10}$)Aryloxyreste oder ($C_7$-$C_{10}$)-Aralkoxyreste sind.

**3.** Verbindungen gemäß Anspruch 1 oder 2, dadurch gekennzeichnet, daß $R^1$ für ein Wasserstoffatom steht.

**4.** Verfahren zur Herstellung der Verbindungen gemäß einem oder mehreren der Ansprüche 1 bis 3, dadurch gekennzeichnet, daß in einer ersten Stufe ein Aldehyd bzw. Keton der allgemeinen Formel II

$X\text{-}CO\text{-}R^1$ (II)

mit einem niedrigsiedenden Alkohol in Gegenwart eines Katalysators und eines wasserentziehenden Mittels in ein Acetal bzw. Ketal der allgemeinen Formel III

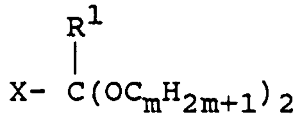

(III)

überführt und dieses dann in einer zweiten Stufe durch Umsetzung mit einem hochsiedenden Alkohol $R^2$-OH und/oder $R^3$-OH in Gegenwart eines Katalysators zu den Verbindungen der allgemeinen Formel I umgewandelt wird.

**5.** Verfahren gemäß Anspruch 4, dadurch gekennzeichnet, daß der niedrigsiedende Alkohol Methanol oder Ethanol ist.

**6.** Verfahren gemäß Anspruch 4 oder 5, dadurch gekennzeichnet, daß der in der ersten Stufe verwendete Katalysator $RhCl_3$(triphos) ist.

**7.** Verfahren gemäß einem oder mehreren der Ansprüche 4 bis 6, dadurch gekennzeichnet, daß der in der ersten Stufe verwendete Katalysator $RhCl_3[CH_3C(CH_2PPh_2)_3]$ ist.

**8.** Verfahren gemäß einem oder mehreren der Ansprüche 4 bis 7, dadurch gekennzeichnet, daß das wasserentziehende Mittel ein dem verwendeten niedrigsiedenden Alkohol entsprechender Orthoameisensäuretrialkylester ist.

**9.** Verfahren gemäß einem oder mehreren der Ansprüche 4 bis 8, dadurch gekennzeichnet, daß der in der zweiten Stufe verwendete Katalysator Kaliumhydrogensulfat ist.

**10.** Verfahren gemäß einem oder mehreren der Ansprüche 4 bis 9, dadurch gekennzeichnet, daß der in der zweiten Stufe verwendete Katalysator ein saures Ionenaustauscherharz ist.

**11.** Positiv arbeitendes strahlungsempfindliches Gemisch mit

a) einer Verbindung, die unter der Einwirkung von aktinischer Strahlung starke Säure bildet,

b) eine Verbindung mit mindestens eine C-O-C-Bindung, die durch die von der Verbindung a) gebildete Säure gespalten werden kann, und

c) ein in Wasser unlösliches, in wäßrig-alkalischer Lösung dagegen lösliches oder zumindest quellbares Bindemittel,

dadurch gekennzeichnet, daß die Verbindung b) eine Verbindung gemäß einem oder mehreren der Ansprüche 1 bis 3 ist.

**12.** Positiv arbeitendes strahlungsempfindliches Gemisch gemäß Anspruch 11, dadurch gekennzeichnet, daß die Verbindung a) unter der Einwirkung von aktinischer Strahlung Sulfonsäure bildet.

**13.** Positiv arbeitendes strahlungsempfindliches Gemisch gemäß Anspruch 11 oder 12, dadurch gekennzeichnet, daß die Verbindung a) einen Anteil von 0,25 bis 25 Gew.-%, bevorzugt 0,5 bis 15 Gew.-%, besonders bevorzugt

1,0 bis 10 Gew.-%, an dem Gesamtgewicht der festen Anteile des Gemisches hat.

**14.** Positiv arbeitendes strahlungsempfindliches Gemisch gemäß einem oder mehreren der Ansprüche 11 bis 13, dadurch gekennzeichnet, daß die Verbindung b) einen Anteil von 1 bis 60, bevorzugt 5 bis 50 Gew.-%, an dem Gesamtgewicht der festen Anteile des Gemisches hat.

**15.** Positiv arbeitendes strahlungsempfindliches Gemisch gemäß einem oder mehreren der Ansprüche 11 bis 14, dadurch gekennzeichnet, daß das Bindemittel c) einen Anteil von 30 bis 95 Gew.-%, bevorzugt 40 bis 90 Gew.-%, besonders bevorzugt 50 bis 85 %, an dem Gesamtgewicht der festen Anteile des Gemisches hat.

**16.** Strahlungsempfindliches Aufzeichnungsmaterial aus einem Substrat und einer darauf befindlichen Schicht, dadurch gekennzeichnet, daß die strahlungsempfindliche Schicht aus einem Gemisch gemäß einem oder mehreren der Ansprüche 11 bis 15 besteht.

## Claims

**1.** A compound of the formula I

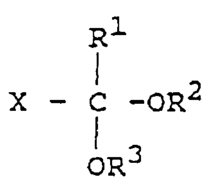 (I)

in which

X

is a phenyl, [1]naphthyl or [2]naphthyl radical each substituted by at least one tert.-butoxycarbonyloxy group and, if appropriate, having one or more than one additional substituent,

$R^1$

is a hydrogen atom, a $(C_1\text{-}C_6)$-alkyl radical, a $(C_6\text{-}C_{10})$-aryl radical or one of the radicals X and

$R^2$ and $R^3$

are identical or different and are a $(C_1\text{-}C_{12})$-alkyl radical in which, if appropriate, up to three methylene groups are replaced by bridge members having at least one hetero atom, such as -O-, -S-, $-NR^4-$, -CO-, -CO-O-, -CO-NH-, -O-CO-NH-, -NH-CO-NH-, -CO-NH-CO-, $-SO_2-$, $-SO_2-O-$ or $-SO_2-NH-$, a $(C_3\text{-}C_{12})$-alkenyl, $(C_3\text{-}C_{12})$-alkynyl, $(C_4\text{-}C_{12})$-cycloalkyl, $(C_4\text{-}C_{12})$-cycloalkenyl or $(C_8\text{-}C_{16})$-aralkyl radical in which, if appropriate, up to three methylene groups of the aliphatic moiety are replaced by bridge members of the abovementioned type and which can be substituted in the aromatic moiety by fluorine, chlorine or bromine atoms or by $(C_1\text{-}C_4)$-alkyl, $(C_1\text{-}C_4)$-alkoxy, nitro, cyano or tert.-butoxycarbonyloxy groups,

$R^4$

being an acyl radical, especially a $(C_1\text{-}C_6)$-alkanoyl radical.

**2.** A compound as claimed in claim 1, wherein the substituents which may additionally be present in the radicals X are fluorine, chlorine, bromine or iodine atoms, nitro, cyano or carboxyl groups, $(C_1\text{-}C_9)$- and particularly preferably $(C_1\text{-}C_6)$-alkyl radicals in which, if appropriate, up to three and particularly preferably up to two methylene groups are replaced by bridge members of the type indicated in claim 1, phenyl radicals which may in turn be substituted, especially by tert.-butoxycarbonyl groups, $(C_1\text{-}C_4)$-alkyl radicals, $(C_1\text{-}C_4)$-alkoxy radicals and/or halogen atoms, and also $(C_8\text{-}C_{12})$- and particularly preferably $(C_8\text{-}C_{10})$-aralkyl radicals in which, if appropriate, up to two methylene groups are replaced by bridge members of the abovementioned type, $(C_6\text{-}C_{10})$-aryloxy radicals or $(C_7\text{-}C_{10})$-aralkoxy radicals.

**3.** A compound as claimed in claim 1 or 2, wherein $R^1$ is a hydrogen atom.

**4.** A process for preparing a compound as claimed in one or more of claims 1 to 3, which comprises converting, in a first stage, an aldehyde or ketone of the formula II

$X\text{-}CO\text{-}R^1$ (II)

by means of a low-boiling alcohol in the presence of a catalyst and of a dehydrating agent into an acetal or ketal of the formula III

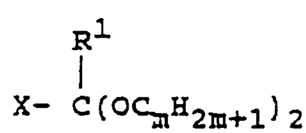 (III)

and then converting the latter, in a second stage, by reaction with a high-boiling alcohol $R^2$-OH and/or $R^3$-OH in the presence of a catalyst into the compound of the formula I.

**5.** The process as claimed in claim 4, wherein the low-boiling alcohol is methanol or ethanol.

**6.** The process as claimed in claim 4 or 5, wherein the catalyst used in the first stage is $RhCl_3$ (triphos).

**7.** The process as claimed in one or more of claims 4 to 6, wherein the catalyst used in the first stage is $RhCl_3[CH_3C(CH_2PPh_2)_3]$.

**8.** The process as claimed in one or more of claims 4 to 7, wherein the dehydrating agent is a trialkyl orthoformate corresponding to the low-boiling alcohol used.

**9.** The process as claimed in one or more of claims 4 to 8, wherein the catalyst used in the second stage is potassium hydrogensulfate.

**10.** The process as claimed in one or more of claims 4 to 9, wherein the catalyst used in the second stage is an acidic ion exchanger resin.

**11.** A positive-working radiation-sensitive mixture with

a) a compound which generates a strong acid under the action of actinic radiation,

b) a compound having at least one C-O-C bond which can be cleaved by the acid generated by the compound a), and

c) a binder which is insoluble in water but soluble or at least swellable in aqueous-alkaline solution,

wherein the compound b) is a compound as claimed in one or more of claims 1 to 3.

**12.** The positive-working radiation-sensitive mixture as claimed in claim 11, wherein the compound a) generates a sulfonic acid under the action of actinic radiation.

**13.** The positive-working radiation-sensitive mixture as claimed in claim 11 or 12, wherein the proportion of compound a) in the total weight of the solid constituents of the mixture is 0.25 to 25% by weight, preferably 0.5 to 15% by weight, and particularly preferably 1.0 to 10% by weight.

**14.** The positive-working radiation-sensitive mixture as claimed in one or more of claims 11 to 13, wherein the proportion of compound b) in the total weight of the solid constituents of the mixture is 1 to 60 and preferably 5 to 50% by weight.

**15.** The positive-working radiation-sensitive mixture as claimed in one or more of claims 11 to 14, wherein the proportion of the binder c) in the total weight of the solid constituents of the mixture is 30 to 95% by weight, preferably 40 to 90% by weight and particularly preferably 50 to 85%.

**16.** A radiation-sensitive recording material composed of a substrate and a layer located thereon, wherein the radiation-sensitive layer comprises a mixture as claimed in one or more of claims 11 to 15.

## Revendications

**1.** Composés de formule générale I

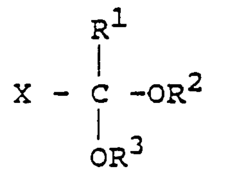

(I)

dans laquelle

X est un radical phényle, [1]-naphtyle ou [2]-naphtyle, lequel est substitué par au moins un groupe tert-butoxycarbonyloxy et lequel présente éventuellement un ou plusieurs substituants supplémentaires;

$R^1$ représente un atome d'hydrogène ou un groupe ($C_1$-$C_6$ alkyle, un groupe ($C_6$-$C_{10}$)aryle ou un radical X; et

$R^2$ et $R^3$ identiques ou différents représentent un radical ($C_1$-$C_{12}$)alkyle dans lequel 1 à 3 groupes méthyles sont éventuellement remplacés par des biradicaux pontants présentant au moins un hétéro atome tel que -O-, -S-, -$NR^4$-, -CO-, -CO-O-, -CO-NH-, -O-CO-NH-, -NH-CO-NH-, -CO-NH-CO-, -$SO_2$-, -$SO_2$-O-, ou -$SO_2$-NH-, un radical ($C_3$-$C_{12}$)alcényle, un radical ($C_3$-$C_{12}$)alcinyle, un radical ($C_4$-$C_{12}$)-cycloalkyle, un radical ($C_4$-$C_{12}$)cycloalcényle ou un radical ($C_8$-$C_{16}$)aralkyle dans lequel 1 à 3 groupes méthyles de la partie aliphatique sont éventuellement remplacés par des biradicaux pontants du type mentionné ci-dessus et lequel peut être substitué sur sa partie aromatique par des atomes de fluor, de chlore ou de brome, par des groupes ($C_1$-$C_4$)alkyle, par des groupes ($C_1$-$C_4$)alcoxy, par des groupes nitro, cyano ou tertbutoxycarbonyloxy; ou

$R^4$ représente un radical acyle, plus particulièrement un radical ($C_1$-$C_6$)alcanoyle.

**2.** Composés selon la revendication 1, caractérisés en ce que les substituants supplémentaires éventuellement présents dans les radicaux X sont choisis parmi les atomes de fluor, de chlore, de brome ou d'iode, les groupes nitro, cyano ou carboxy, les radicaus $(C_1-C_9)$alkyle, préférablement $(C_1-C_6)$alkyle, dans lequels de 1 à 3, préférablement de 1 à 2 groupes méthyles sont remplacés par les biradicaux pontants mentionnés à la revendication 1, des radicaux phényles qui peuvent être eux-même substitutés, plus particulièrement avec des groups tert-butoxycarbonyle, des radicaux $(C_1-C_4)$alcyle, des radicaux $(C_1-C_4)$alcoxy et/ou des atomes d'halogène, ainsi que des radicaux $(C_8-C_{12})$-aralkyle, préférablement $(C_8-C_{10})$alralkyle dans lesquels de 1 à 2 groupes méthyles sont éventuellement remplacés par les biradicaux pontants mentionnés à la revendication 1, des radicaux $(C_6-C_{10})$aryloxy ou des radicaux $(C_7-C_{10})$aralcoxy.

**3.** Composés selon la revendication 1 ou la revendication 2, caractérisés en ce que $R^1$ représente un atome d'hydrogène.

**4.** Procédé pour la préparation des composés selon l'une quelquonque des revendications 1 à 3, caractérisé en ce que, dans une première étape, on convertit un aldéhyde ou une cétone de formule générale II

$X\text{-}CO\text{-}R^1$ (II)

en un acétal ou cétal de formule générale III

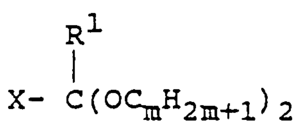

(III)

à l'aide d'un alcool de bas point d'ébullition en présence d'un catalyseur et d'un agent capteur d'eau et ensuite, dans une deuxième étape, on transforme ce composé en un composé de formule générale I par réaction avec un alcool $R^2$-OH et/ou $R^3$-OH de haut point d'ébullition en présence d'un catalyseur.

**5.** Procédé selon la revendication 4, caractérisé en ce que l'alcool de bas point d'ébullition est du méthanol ou de l'éthanol.

**6.** Procédé selon la revendication 4 ou la revendication 5, caractérisé en ce que dans la première étape on utilise, comme catalyseur, du $RhCl_3$(triphos).

**7.** Procédé selon l'une quelconque des revendications 4 à 6, caractérisé en ce que dans la première étape on utilise comme catalyseur $RhCl_3[CH_3C(CH_2PPh_2)_3]$.

**8.** Procédé selon l'une quelquonque des revendications 4 à 7, caractérisé en ce que l'agent capteur d'eau est l'orthoformiate de trialkyle correspondant à l'alcool de bas point d'ébullition utilisé.

**9.** Procédé selon l'une quelconque des revendications 4 à 8, caractérisé en ce que, dans la deuxième étape, on utilise comme catalyseur l'hydrogénosulfate de potassium.

**10.** Procédé selon l'une quelquonque des revendications 4 à 9, caractérisé en ce que, dans la deuxième étape, on utilise comme catalyseur une résine acide échangeuse d'ions.

**11.** Composition photosensible travaillant en positif constitué de:

a) un composé, qui sous l'action d'un rayonnement actinique conduit à un acide fort,

b) un composé présentant au moins une liaison C-O-C dissociable sous l'action de l'acide formé à partir du composé a); et

c) un liant insoluble dans l'eau mais en revanche soluble ou au moins gonflant dans une solution aqueuse alcaline, caractérisée en ce que le composé b) est un composé selon l'une quelquonque des revendications 1 à 3.

**12.** Composition photosensible travaillant en positif selon la revendication 11, caractérisée en ce que le composé a) conduit à un acide sulfonique sous l'action d'un rayonnement actinique.

**13.** Composition photosensible travaillant en positif selon la revendication 11 ou la revendication 12, caractérisée en ce que le composé a) représente de 0,25 à 25 % en poids, de préférence de 0,5 à 15 % en poids, plus préférablement de 1.0 à 10 % en poids, du poids total de la fraction solide de la composition.

**14.** Composition photosensible travaillant en positif selon l'une quelquonque des revendications 11 à 13, caractérisé en ce que le composé b) représente del à 60 % en poids, de préférence de 5 à 50 % en poids, du poids total de la fraction solide de la composition.

**15.** Composition photosensible travaillant en positif selon l'une quelquonque des revendications 11

à 14, caractérisée en ce que le composé c) représente de 30 à 95 % en poids, de préférence de 40 à 90 % en poids, plus préférablement de 50 à 85 % en poids, du poids total de la fraction solide de la composition.

**16.** Matériau d'enrigistrement photosensible constitué d'un substrat et d'une couche disposée sur ledit substrat, caractérisé en ce que la couche photosensible et constituée d'une composition selon l'une quelquonque des revendications 11 à 15.